# EUROPEAN PATENT SPECIFICATION

(11) **EP 4 450 110 B1**
(45) Date of publication and mention of the grant of the patent: **02.07.2025**
(21) Application number: 24171366.8
(22) Date of filing: 19.04.2024
(51) Int. Cl.: A61M 25/00, A61M 25/01, A61B 17/12, A61B 17/22, A61M 25/10, A61B 90/00

(54) **DEVICE FOR INFUSION OF PHARMACOLOGIC AGENTS AND THROMBUS ASPIRATION IN ARTERY**
VORRICHTUNG ZUR INFUSION VON PHARMAKOLOGISCHEN WIRKSTOFFEN UND THROMBUSASPIRATION IN ARTERIEN
DISPOSITIF DE PERFUSION D'AGENTS PHARMACOLOGIQUES ET D'ASPIRATION DE THROMBUS DANS UNE ARTERE

(30) Priority: 19.04.2023 US 202363496978 P
(43) Date of publication of application: 23.10.2024
(73) Proprietor: Valorisation Recherche HSCM, S.E.C, Quebec, QC G1P 4P5 (CA)
(72) Inventor: CHARRON, Thierry, MONT ST-HILAIRE, J3H 6J3 (CA); LEBLANC, Aimé-Robert, MONTREAL, H4J 1C5 (CA); ROUSSEAU, Guy, ILE BIZARD, H9E 1R9 (CA); CHU, Boby, LASALLE, H8P 3K2 (CA); BENARD, Benjamin, MONTREAL, H2K 3H5 (CA); DAVID, Lianna, MONTREAL, H1R 2M5 (CA); ROMERO VASQUEZ, Andres, MONTREAL, H3G 2M2 (CA)
(74) Representative: Regimbeau

(56) References cited:
- CN-A- 113 546 281
- US-A1- 2006 161 103
- US-A1- 2015 190 558

## Description

### FIELD OF THE DISCLOSURE

The present application pertains to a catheter assembly for the infusion of pharmacologic agents and thrombus aspiration in atherosclerotic vascular disease.

### BACKGROUND OF THE ART

A prevalent cause of adult mortality in developed countries is atherosclerotic vascular disease.

For example, occlusion of a coronary artery subsequent to a plaque rupture remains one of the most frequent causes of myocardial infarction.

To treat such ailment, percutaneous coronary intervention is an efficient reperfusion method when performed promptly. Yet, the opening of coronary arteries is an invasive and risky procedure. Despite efficient and prompt intervention, some patients will present a condition known as no-reflow in which, in spite of the fact that the coronary artery is opened without residual stenosis, myocardial perfusion is diminished. No-reflow may be caused by two distinct phenomenon. A first one is the distal embolization of microparticles in blood vessels. A second phenomenon is reperfusion injury, which increases myocardial damage despite restoring blood flow in the artery.

Aspiration catheters are efficient in removing blood clots from arteries in the myocardial infarction status, in an effort improve the blood flow in the infarction zone. However, despite their efficiency, such catheters do not necessarily prevent the distal embolization of microparticles subsequent to their use. Also, such catheters do not allow drug infusion distally to the occlusion prior to thrombus aspiration to limit reperfusion injury.

Document US 2015/190558 discloses a catheter assembly.

### SUMMARY OF THE APPLICATION

It is therefore an aim of the present disclosure to provide a novel device for infusing pharmacologic agents and/or performing thrombus aspiration in atherosclerotic vascular disease.

The present disclosure provides also a non-claimed method for infusing pharmacologic agents and/ or performing thrombus aspiration in artherosclerotic vascular disease.

Therefore, in accordance with an aspect of the present disclosure, there is provided a catheter assembly comprising a distal shaft having a guide lumen and an infusion lumen; a proximal shaft connected to the distal shaft, the proximal shaft defining an aspiration lumen opening at or near the distal shaft, and an inflating lumen; an inflatable member at a distal end of the inflating lumen, the inflatable member configured to inflate upon receiving a fluid from the inflating lumen; a stylet tube extending along the aspiration lumen and in fluid communication with the infusion lumen, the stylet tube configured to supply the infusion lumen with a pharmacologic agent; wherein the catheter assembly is configured to be displaced percutaneously in an artery by the guide lumen moving along a guide wire.

Further in accordance with the aspect, for instance, a catheter hub may be at a proximal end of the proximal shaft.

Still further in accordance with the aspect, for instance, the catheter hub is in fluid communication with the stylet tube, with the aspiration lumen and with the inflating lumen.

Still further in accordance with the aspect, for instance, the catheter hub has a port dedicated to the inflating lumen.

Still further in accordance with the aspect, for instance, a three-way valve may be on the port dedicated to the inflating lumen.

Still further in accordance with the aspect, for instance, radio-opaque markers may be provide dat or near opposed ends of the infusion lumen.

Still further in accordance with the aspect, for instance, the stylet is removable from the aspiration lumen prior to aspiration.

Still further in accordance with the aspect, for instance, the distal shaft has an aspiration port in fluid communication with the aspiration lumen.

Still further in accordance with the aspect, for instance, a proximal end of the guide lumen is at the distal shaft.

Still further in accordance with the aspect, for instance, a guide wire may be part of the catheter assembly.

In accordance with another aspect of the present disclosure, which is not covered by the claims, there is provided a use of a catheter assembly for removing a thrombus from an artery, comprising: a guide wire for being percutaneously positioned into an artery toward the thrombus; a distal shaft of a catheter for being moved along the guide wire until a tip of an infusion lumen of the distal shaft passes through the thrombus to a distal location; an inflatable member on the catheter and in fluid communication with an inflating lumen of the catheter for inflating and blocking the artery proximally of the thrombus; an aspiration lumen in the catheter extending into a proximal shaft of the catheter to reach a position proximal to the thrombus, for aspiring the thrombus; and a stylet removably in the aspiration lumen of proximal shaft and in fluid communication with the infusion lumen for infusing at least one pharmacologic agent distally to the thrombus via the infusion lumen.

In accordance with another aspect of the present application, which is not covered by the claims, there is provided a method for treating an artery having a thrombus comprising: percutaneously positioning a guide wire into an artery toward the thrombus; moving a distal shaft of a catheter along the guide wire until a tip of an infusion lumen of the distal shaft passes through the thrombus to a distal location, while an inflatable member and an aspiration lumen extending into a proximal shaft of the catheter are proximal to the thrombus; and infusing at least one pharmacologic agent distally to the thrombus via the infusion lumen.

Further in accordance with the aspect, for instance, inflating the inflatable member may occur proximally to the thrombus. In the present disclosure, proximally may refer to positions upstream of a thrombus relative to blood flow, while distal may refer to positions downstream of a thrombus relative to blood flow.

Still further in accordance with the aspect, for instance, inflating the inflatable member proximally to the thrombus is performed prior to infusing at least one pharmacologic agent distally to the thrombus.

Still further in accordance with the aspect, for instance, inflating the inflatable member is performed during fluoroscopy to monitor the inflating.

Still further in accordance with the aspect, for instance, further an aspiration may be performed via the aspiration lumen to aspire the thrombus.

Still further in accordance with the aspect, for instance, positioning the catheter and passing of the tip are done by moving the catheter over a guide wire.

Still further in accordance with the aspect, for instance, moving the distal shaft of the catheter over the guide wire includes only the distal shaft being engaged onto the guide wire by a guide lumen thereof.

Still further in accordance with the aspect, for instance, infusing at least one pharmacologic agent includes infusing the pharmacologic agent in a tube of a stylet extending to the infusion lumen.

Still further in accordance with the aspect, for instance, infusing the pharmacologic agent in a tube of a stylet extending to the infusion lumen includes infusing the pharmacologic agent in the tube of the stylet passing through the aspiration lumen.

Still further in accordance with the aspect, for instance, the stylet is optionally from the aspiration lumen prior to using the aspiration lumen to aspire the thrombus.

### BRIEF DESCRIPTION OF THE DRAWINGS

Fig. 1 is a schematic view of a catheter assembly for infusing a pharmacologic agent and for thrombus aspiration in accordance with an embodiment of the present disclosure;
Fig. 2 is a schematic view of the catheter assembly of Fig. 1, with a stylet tube therein;
Fig. 3 is a schematic view of the stylet tube of Fig. 2;
Fig. 4 is a schematic cross-sectional view of a proximal shaft of the catheter assembly of Fig. 1;
Fig. 5 is a sectional cross-sectional view of the proximal shaft of the catheter assembly of Fig. 2, showing the stylet tube;
Fig. 6 is cross-sectional view of a delivery tip of a distal shaft of the catheter assembly of Fig. 1;
Fig. 7 is a flowchart of a method for infusing a pharmacologic agent and performing thrombus aspiration in accordance with yet another embodiment of the present disclosure;
Figs. 8A to 8C illustrate an exemplary sequence of maneuvers at a central hub of the catheter assembly of an embodiment of the present disclosure, to prepare a balloon for being inflated;
Figs. 9A and 9B illustrate a flush of a stylet and of an aspiration lumen of the catheter assembly of an embodiment of the present disclosure;
Fig. 10 illustrate an arrangement of syringes at the central hub of the catheter assembly of an embodiment of the present disclosure, to inflate the balloon; and
Fig. 11 depict the central hub of the catheter assembly of an embodiment of the present disclosure, prior to aspiration.

### DESCRIPTION OF THE EMBODIMENTS

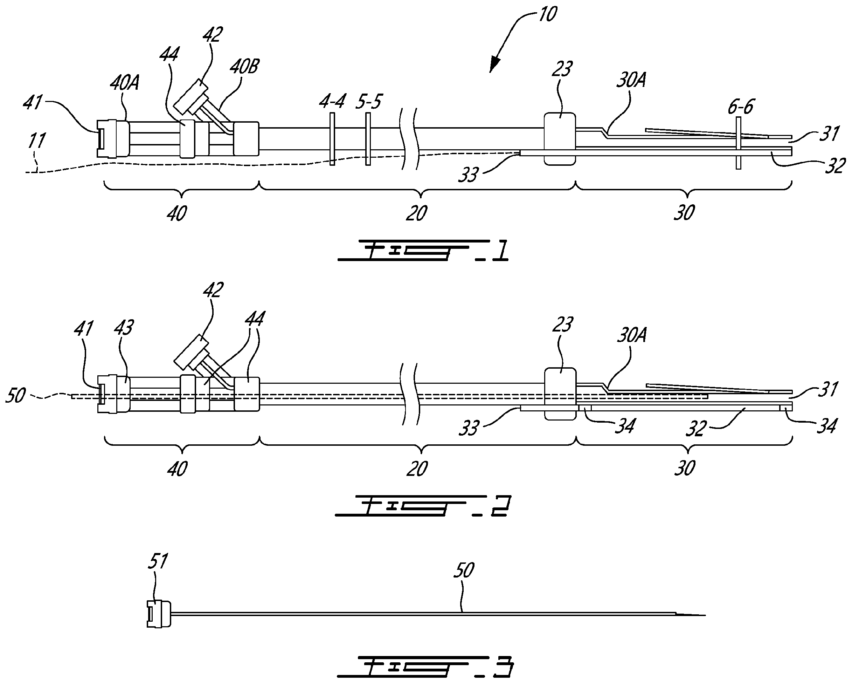

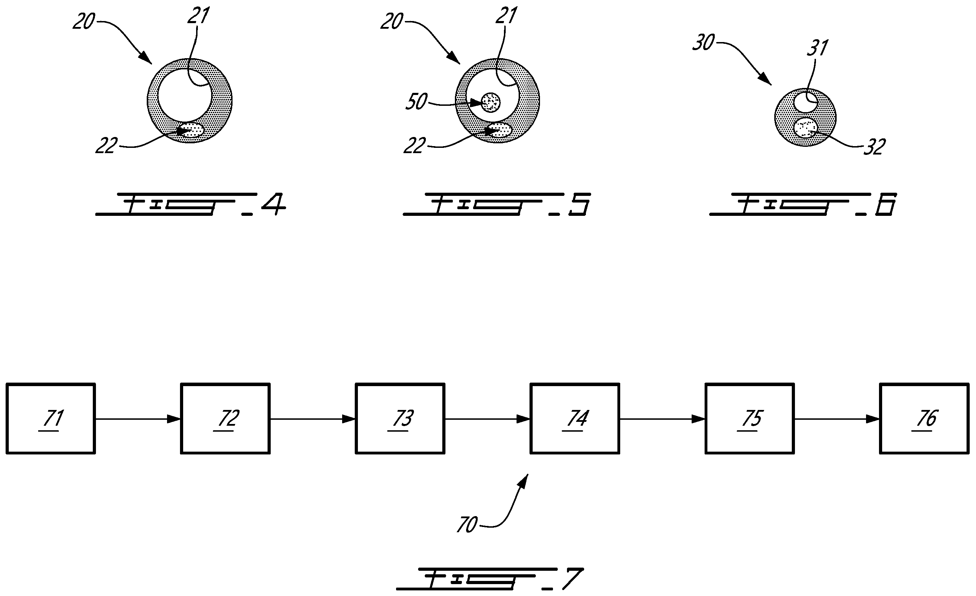

Referring to Figs. 1 and 2, a catheter assembly in accordance with the present disclosure is generally shown at 10. The catheter assembly 10 may be used for the infusion of pharmacologic agents and for thrombus aspiration in accordance with the methodology described for method 70 of Fig. 7 as described hereinafter, through the method 70 and the catheter assembly 10 may not be mutually exclusive. In some variants, the method 70 may be used with a catheter assembly differing the catheter assembly 10, and the catheter assembly 10 may be used with a method differing from the method 70 of Fig. 7.

The catheter assembly 10 is introduced over a guide wire 11 that is inserted into any appropriate vessel of the vascular system, such as the coronary artery, with a view to attend to a thrombus. As detailed hereinafter, the catheter assembly 10 has a hydrophilic surface, so as to slide within the artery with reduced invasiveness.

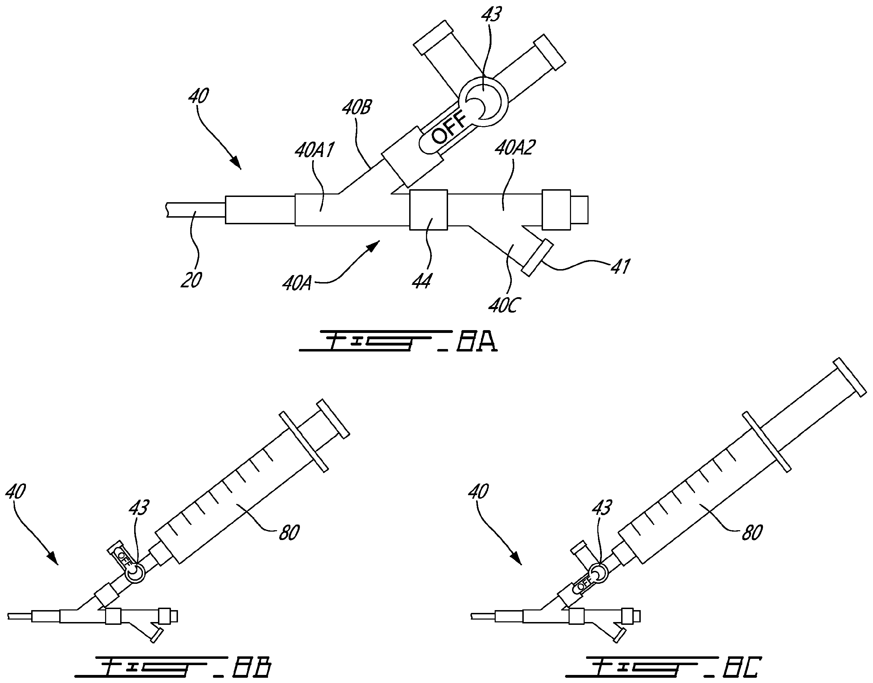

The catheter assembly 10 may have one or more of the guide wire 11, though the guide wire 11 may be an add-on that is not part of the catheter assembly 10, a proximal shaft 20, a distal shaft 30, a catheter hub 40, and/or a stylet 50, though the stylet 50 may not be part of the catheter assembly 10. Other components may be part of the catheter assembly 10, such as any of those described herein through Figs. 8A-11. The guide wire 11 is the guide upon which parts of the catheter assembly 10 will be slid or guided in order to reach a target zone.

The catheter assembly 10 includes the proximal shaft 20, the distal shaft 30 and/or may include the catheter hub 40. It may also be said that the guide wire 11 may be part of the catheter. The proximal shaft 20 is between the distal shaft 30 and the catheter hub 40, and serves as a canal for fluid displacement (and possibly solids), among other functions, in a distal direction and/or proximal direction. The proximal shaft 20 may be the longest component of the catheter (e.g. depending on whether the guide wire 11 is part or not of the catheter).

Figs. 1 and 2 are schematic views and may not be representative of the length of the proximal shaft 20 relative to the distal shaft 30 and catheter hub 40. The distal shaft 30 is at the distal end of the proximal shaft 20 and may be one of the output ends of the catheter. The catheter hub 40 may be located outside of the body during a procedure, while the proximal shaft 20 is mostly in the body - it may gradually be inserted into the body -, and the distal shaft 30 is within the body. The catheter hub 40 is the interface that may be manipulated by an operator, to perform infusion, inflating and aspiration, for example. The stylet 50 may be used in the infusion of a pharmacologic agent in the human body, and may also be referred to as a tube. In a variant, the stylet 50 provides some rigidity to the catheter assembly 10, and may be optionally used to transmit a push force when positioning the catheter device in the vessel. As a possibility, the stylet 50 is a one-time use device.

Referring to Figs. 1 and 2, the proximal shaft 20 is shown as having an elongated body, that is merely shown schematically but that may be substantially longer than what is shown in Figs. 1 and 2. The proximal shaft 20 may have a monoblock body, for instance that may be extruded. Other constructions include a combination of materials, braiding, etc. The proximal shaft 20 may include an outer sheath that incorporates therein different tubes that may define different lumens, as described below, or may be monoblock and define one or more of these lumens. The proximal shaft 20 may also have different constructions, such as by forming an outer jacket supporting different tubes, that will define different types of lumens. The following description pertains to a monoblock body for the shaft portion of the proximal shaft 20, though other types of constructions are considered, and are suggested as well where applicable.

Referring to Figs. 4 and 5, the proximal shaft 20 defines an aspiration lumen 21, and an inflating lumen 22. An inflatable member 23, such as a balloon, is provided at the distal end of the proximal shaft 20 and is in fluid communication with the inflating lumen 22, such that the balloon 23 may be inflated. While the inflatable member 23 may be positioned at or near the distal end of the proximal shaft 20, it may also be located at a junction between the proximal shaft 20 and the distal shaft 30, and/or may be part of the distal shaft 30. The inflatable member 23 may be any suitable type of inflatable material, such as a balloon. According to an embodiment, the inflatable member 23 is made of a substantially compliant membrane, so as to distribute inflating pressure uniformly on the inner surface of the artery or other vessel.

The aspiration lumen 21 is open-ended at the distal end of the proximal shaft 20. In an embodiment, the aspiration lumen 21 is part of an aspiration tube. Such an aspiration tube may be made from a combination of layers, such as polytetrafluoroethylene, a stainless steel braid (e.g., 0.0005" (0.013 mm) thickness for 0.005" (0.13 mm) width of flat wire with 44 pics/in (17 pics/cm)), and a polyether block amide lumen (e.g., Pebax^{™}. 6333). As an example, the aspiration lumen 21 may have an inner diameter of 0.040" (1.02 mm), though this is merely given as an example. A typical inner diameter of the aspiration lumen 21 ranges from 0.030" (0.76 mm) to 0.045" (1.14 mm), though it may be outside this range. If an aspiration tube is present, it may have a wall thickness of 0.0025" (0.063 mm) and an outer diameter of 0.045" (0.11 mm), again with these values merely provided as examples. Other dimensions are considered as well. According to an embodiment, a polytetrafluoroethylene (PTFE) tube is placed over a mandrel also covered with PTFE, and the stainless steel braid is then positioned on the PTFE.

As observed in Fig. 5, the aspiration lumen 21 hosts the stylet 50, such that the pharmacological agent may be infused through the aspiration lumen 21, via the stylet 50, though the infusion lumen of the stylet 50 may be isolated from the aspiration lumen 21. During use, the open distal end of the aspiration lumen 21 is proximally behind the open distal end of the stylet 50, and is hence proximally located in the artery relative to a position of the inflatable member 23.

The inflating lumen 22 may or may not be part of an inflating tube. The inflating lumen 22 opens into the inflatable member 23 at the front end of the catheter assembly 10, for injection of a fluid into the inflatable member 23 via the inflating lumen 22. According to an embodiment, the inflating lumen 22 is part of a tube that may be made from a single extruded material. However, a combination of layers may be considered as well, such as high-density polyethylene (e.g., HDPE LR734, 25%) and/or polyether block amide (e.g., Pebax^{™} 7233, 75% of outer layer) and/or Plexar^{™} (e.g., middle layer of Plexar^{™} 3080). As an example, the inflating lumen 22 may have an inner diameter of 0.018", though this is merely given as an example. A typical inner diameter of the inflating lumen 22 ranges from 0.001 to 0.038". (0.025 to 0.97 mm) If present, the inflating tube may have a wall thickness of 0.0020" (0.051 mm) and an outer diameter of 0.022" (0.56 mm), again with these values merely provided as examples. Other dimensions are considered as well. Any appropriate type of fluid may be used. For instance, a mixture of saline and iodine contrast is commonly used for inflating balloons in percutaneous coronary intervention. The fluid used to inflate an inflatable member may have suitable contrast to be detected via radiographic imaging.

Referring to Figs. 1 and 2 and 6, the distal shaft 30 is shown as having a body tapering from a proximal end toward a distal end, to facilitate its displacement in a vessel. The distal shaft 30 may also have a monoblock body, for instance that may be extruded, but this is only an option as the distal shaft 30 may be constituted of more than one component. For example, the distal shaft 30 may also have different constructions, such as by forming an outer jacket supporting different tubes, that will define different types of lumens.

The distal shaft 30 may have an aspiration port 30A. In a variant, the aspiration lumen 21 in the proximal shaft 20 is in fluid communication with the body of the distal shaft 30 and thus with the aspiration portion 30A, with the aspiration port 30A opening into the vessel. Accordingly, the aspiration port 30A may be a suction port of the catheter. The distal shaft 30 may then taper to form a smaller cross-section that may be passed through a thrombus. In a variant, the aspiration lumen 21 in the proximal shaft 20 opens directly into the vessel, such that the distal shaft 30 is not part of the aspiration step.

The distal shaft 30 also has an infusion lumen 31, that is used to deliver the pharmacologic agent at the distal end of the catheter. The infusion lumen 31 may be a passage of the stylet 50, or may be in fluid communication with the distal end of the stylet 50 so as to receive the pharmacological agent and deliver same to the infusion target site. As observed, the infusion lumen 31 may have a funneling geometry in a distal direction. In Fig. 6, this scenario is shown, i.e., the stylet 50 is fluidly connected to the infusion lumen 31, for the pharmacologic agent to travel through the infusion lumen 31 and out of the distal tip of the distal shaft 30. The funnel geometry of the infusion lumen 31 is optional, but is well suited to ensure that the stylet 50 is in fluid communication with and delivers its pharmacologic agent to the infusion lumen 31.

The distal shaft 30 further includes a guide lumen 32 configured to be mounted onto the guide wire 11, for relative movement between the guide wire 11 and the distal shaft 30. In a variant, the guide lumen 32 opens at the distal end of the distal shaft 30. In a variant, the guide lumen 32 ends at or near the proximal end of the distal shaft 30, though it may also extend to part of the proximal shaft 20, at an exit port 33. In a variant, the guide lumen 32 has a length of about 6 inches ± 2 inches (about 15 cm ± 5 cm). Hence, the catheter may only have its distal shaft 30 mounted to the guide wire 11, thereby limiting friction, and facilitating displacement. Hence, the catheter may slide along the guide wire 11 during its insertion in the vessel. The distal shaft 30, that may also be part of the outer jacket of the catheter 10, may have an hydrophilic surface, tapering in a distal direction. According to an embodiment, the distal shaft 30 is made of polyether block amide (e.g., Pebax^{™} 5533).

Markers 34 of detectable material may be provided at one or both extremities of the distal shaft 30 for localization thereof, such as at the distal tip of infusion lumen 31. For instance, radio-opaque material may be used for fluoroscopic localization, such as platinum with an iridium content (e.g., 10%). Alternatives are considered as well. According to an embodiment, the markers 34 have an inner diameter of 0.023" (0.58 mm), a wall of 0.001" (0,025 mm) and an outside diameter of 0.025" (0.63 mm). These values are merely provided as examples.

Referring to Figs. 1 and 2, the catheter hub 40 is shown. The proximal shaft 20 may emerge out of the human body, such that the open external ends of the aspiration lumen 21 and inflating lumen 22 are outside of the body, so as to be connected to appropriate means, for example via the catheter hub 40. The catheter hub 40 may have a central tubular portion 40A with a branch portion 40B diverging from the central tubular portion 40A. Other configurations are considered such as a single tubular portion with separate ports, a Y-shaped body, etc. In a variant, the central tubular portion 40A defines a flush port 41 through which suction will be exerted on the catheter, during aspiration. The flush port 41 may therefore be in fluid communication with the aspiration lumen 21. The branch portion 40B may define the inflating port 42, and may thus be in fluid communication with the inflating lumen 22, so as to direct fluid into the inflatable member 23. The flush portion 41 may optionally be provided with a valve 43 to control the suction effect. In a variant, the valve 43 is not part of the catheter assembly 10. In a variant, the stylet 50 is fitted into the catheter via the central tubular portion 40A, though other arrangements are connected.

Collets 44 may be provided to interconnect the hub 40 with the proximal shaft 20, the collets 44 contributing to maintaining the fluid communication between components, as described above. If present, the tubes associated with the lumens 21 and/or 22, and the stylet 50 may be equipped with suitable connectors (e.g., luers) for connection to the various devices (e.g., syringe). For example, one such connector is shown at 51 at the distal end of the stylet 50. The stylet 50 may thus be connected to a source of pharmacologic agents, such as a syringe, etc, for the controlled infusion of pharmacologic agents passing through the catheter of the catheter assembly 10. As mentioned above, the inflating lumen 22 is connected to a source of fluid for pressurization of the inflatable member 23.

Referring to Figs. 8A to 8C, another embodiment of the catheter hub 40 is shown. In Figs. 8A to 8C, the catheter hub 40 may have a central tubular portion 40A with a branch portion 40B diverging from the central tubular portion 40A, and another branch portion 40C also diverging from the central tubular portion 40A. In a variant, the central tubular portion 40A defines at its proximal end the injection port, or at the proximal end of part 40A1. The branch portion 40B may define the inflating port 42, and may thus be in fluid communication with the inflating lumen 22, so as to direct fluid into the inflatable member 23. The branch portion 40C may be the flush port 41 through which suction will be exerted on the catheter, during aspiration. The flush port 41 may therefore be in fluid communication with the aspiration lumen 21. The flush portion 41 may optionally be provided with a valve 43 to control the suction effect, the valve being for example a three-way valve such as a three-way stopcock, as an example among others. In a variant, the valve 43 is not part of the catheter assembly 10. In a variant, the stylet 50 is fitted into the catheter via the free end of the central tubular portion 40A, though other arrangements are connected.

Collet 44 may be provided to interconnect the part 40A1 of the hub 40 featuring the branch portion 40B with the part 40A2 of the hub featuring the branch portion 40C. The stylet 50 may be accessed when separating part 40A1 from part 40A2, as an example. Other collets may be present, such as to connect the central tubular portion 40A to the proximal shaft 20, for example. The collet 44 contributes to maintaining the fluid communication between components, as described above. If present, the tubes associated with the lumens 21 and/or 22, and the stylet 50 may be equipped with suitable connectors (e.g., luers) for connection to the various devices (e.g., syringe).

The aspiration lumen 21, such as through the flush portion 41, is connected to an aspiration mechanism, such as a vacuuming syringe or the like. Accordingly, an aspiration action performed at the external end of the catheter causes an aspiration at the open internal end of the aspiration lumen 21 (e.g., aspiration port 30A), such as at the distal end of the proximal shaft 20 or at an outlet end within the distal shaft 30. As the open internal end of the aspiration lumen 21 is positioned adjacent to the blood clot in the thrombus aspiration application, the blood clot is vacuumed away from the artery via the aspiration lumen 21.

Any appropriate material may be used for the shafts 20 and 30, and/or tubes therein. According to other embodiments, the tubes and/or the stylet 50 is/are, for instance, made of polyimide of medical grade, or any other relatively compliant material. One material that may be used for the shafts 20 and/or 30 and/or stylet 50 is braided reinforced polyimide, to ensure that the aspiration lumen 21 retains its structurally integrity despite the vacuuming pressure, and to maintain the shape during insertion in the vessel, to avoid kinking. During manufacturing, an outer jacket making up the external body of the proximal shaft 20 may be slid over the tubes that would define the aspiration lumen 21 and/or the inflating lumen 22. With tubes fixed and aligned with an outer jacket thereon, the assembly may be passed through a heated die, with the outer jacket fusing all together. Hence, these components are integrally connected to one another, if not monoblock.

Contemplated dimensions are set forth above, by way of example, and for illustrative purposes.

It is understood that the dimensions may be greater or smaller than those set forth above.

Now that the catheter assembly 10 has been described, its non-claimed use in a thrombus aspiration application is described, according to the method 70 of Fig. 7. The non-claimed method 70 may thus be performed by the catheter assembly 10, or by other catheter assemblies to infuse pharmacologic agents and perform thrombus aspiration. In a variant, the method 70 is performed on a cadaver, a model, or on a non-human animal.

According to 71, with the thrombus (a.k.a., blood clot, plaque rupture, artery occlusion) being localized, a distal end of the guide wire 11 is percutaneously inserted onto a blood vessel (e.g., coronary artery), and is moved up to the thrombus. As an option, the guide wire 11 is moved through the thrombus. A proximal end of the guide wire 11 may be out of the body.

According to 72, the catheter of the catheter assembly 10 is percutaneously inserted into the vessel proximally of the occlusion, to be directed distally in the vessel toward the thrombus, as being guided onto the guide wire 11. In the case of the catheter assembly 10, it is the distal shaft 30 that is mounted onto the guide wire 11 outside of the body, via the guide lumen 32, to then be inserted into the vessel as slid along the guide wire 11. The stylet 50 may be used to push the catheter into position, the stylet 50 having been assembled prior to step 72 (for example, as assembled), the stylet 50 being in fluid communication with the infusion lumen 31. It may also be possible to insert the stylet 50 after the combination of the distal shaft 30 and proximal shaft 20 have being guided to the thrombus by moving onto the guide wire 11.

According to 73, a distal tip of the distal shaft 30 of the catheter assembly is directed through the blood clot. The infusion lumen 31 at the end of the distal shaft 30 has thus been guided along the guide wire in an over-the-wire or monorail fashion through the occlusion. Therefore, the tip of the infusion lumen 31 of the catheter is distal of the blood clot after insertion. In a variant, the distal shaft 30 may be localized by the presence of radio-opaque markers 34. Fluoroscopic imagery or any other appropriate type of imaging may be used to locate the infusion tube relative to the thrombus in the treated vessel.

According to 74, an inflatable member on the catheter assembly is inflated proximally of the blood clot. If the catheter assembly 10 is used, the inflatable member 23 is inflated via the inflating lumen 22.

According to 75, a pharmacologic agent (e.g., adenosine) is infused into the infarction zone distally of the occlusion. As the vessel is blocked by the occlusion and the inflated member 23, the pharmacologic agent will diffuse and be absorbed distally to the blood clot. If the catheter assembly 10 is used, the infusion is performed via the stylet 50, that has been inserted into the proximal shaft 20. It may be required that the stylet 50 be slid into the aspiration lumen 21 and displaced in a distal direction, until the distal tip of the stylet 50 reaches the infusion lumen 31. In a variant, as mentioned above, the stylet 50 is pre-positioned in fluid communication with the infusion lumen 31 such that the stylet 50 is moved into position in the vessel concurrently with the positioning of the catheter. This may consequently ensure that the stylet 50 is in suitable fluid communication with the infusion lumen 31 of the distal shaft 30, for delivery of the pharmacologic agent. The infusion of the pharmacologic agent may also be performed prior to the inflation of the inflatable member 23.

According to 76, the blood clot is vacuumed out of the artery so as to open the artery. The aspiration lumen 21 of the catheter assembly 10 is distally positioned relative to the inflatable member 23, such that the inflatable member 23 may not interfere with the vacuuming. In an embodiment, to increase the efficiency of the vacuuming, the stylet 50 is removed from the aspiration lumen 21, prior to the vacuuming. Thus, the aspiration lumen 21 may serve as aspiration passage and as passage for the stylet 50 for the pharmacologic agent. Stated differently, the aspiration lumen 21 may serve for fluid movement in a distal direction, and in proximal direction. This may be simultaneous and/or sequential.

In one non-claimed embodiment, the aspiration is initiated while the inflatable member 23 is inflated. This causes a relative negative pressure between the tip of the aspiration lumen 21 or suction portion 30A and the thrombus. The aspiration may also be initiated after deflation of the inflatable member 23. The deflation may also occur during the aspiration.

Subsequently, the various steps for terminating the intervention are performed, including the removal of the various units of the catheter assembly. For example, the balloon 23 may be deflated if it hasn't yet been deflated. The catheter may then be slid out of the vessel, as moved along the guide wire 11. The guide wire 11 may be the last remaining component in the vessel. It is pointed out that the method 70 may be performed according to any suitable sequence.

With reference to Figs. 8A-8C, some steps may be done before or during the method 70. The balloon 23 (Fig. 1) may be prepared for use by attaching a three-way stopcock to the inflating port 42 of the catheter hub 40. The inflating lumen 22 and a syringe 80 may have been primed beforehand. The syringe 80 may be a larger syringe, i.e., one with a greater volume of solution (e.g., 10 ml). The three-way valve 43 may have its the vertical port in the off position as in Fig. 8A. The syringe 80 may be attached to the valve 43, at the proximal port as shown in Fig. 8B. To do so, the syringe 80 may have its plunger partially or fully depressed to the proximal port of the three-way valve 43. Referring to Fig. 8C, the plunger of the syringe 80 may be pulled, and while maintaining the plunger in place, the valve 43 may be turned counter-clockwise to put the distal port in the OFF position thereby creating a vacuum inside the balloon 23. The syringe 80 may be detached after use.

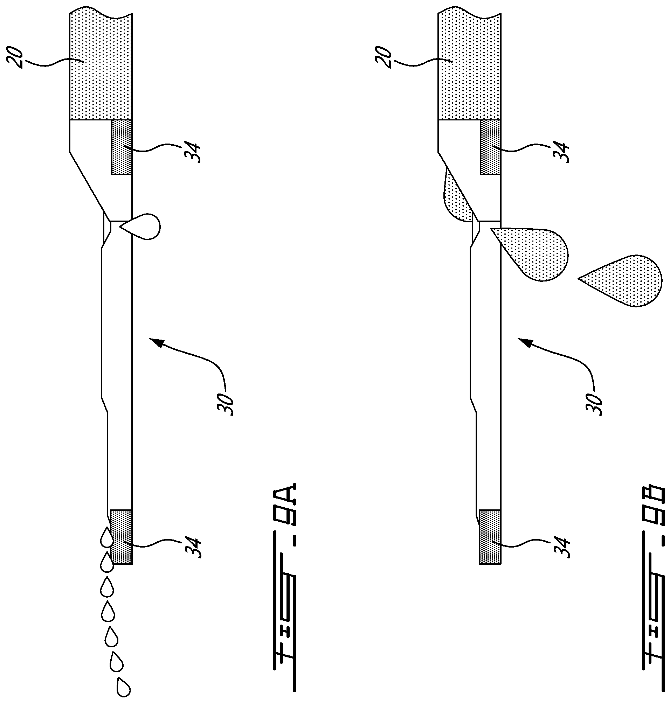

The stylet 50, installed in the catheter assembly, may be flushed by attaching a syringe containing a liquid such as heparinized saline to the injection port at the free end of the central tubular portion 40A (if it is located there) of the catheter hub 40. The plunger of the syringe may be depressed until a jet of fluid exits the distal tip of the catheter and droplets form at the back of the distal tip as in Fig. 9A. The catheter may also be flushed by attaching a syringe to the flush port 41 of the catheter and depressing the plunger until fluid exits the aspiration tip as in Fig. 9B. The syringe may be detached after use.

The catheter may be used with balloon inflation accessories. The balloon inflation accessories may be set up by connecting the syringe 80 filled with a liquid, such as at least 5 mL of a 50:50 v/v saline-contrast mixture (as an example among others), to the proximal port of the three-way valve 43 and depressing the plunger of the syringe 80 to purge air out of the vertical port of the three-way valve 43. Another syringe 81, such as one with a lesser volume, may also be filled with a fluid, such as 50:50 v/v saline-contrast mixture (as an example) to the vertical port of the three-way valve 43.

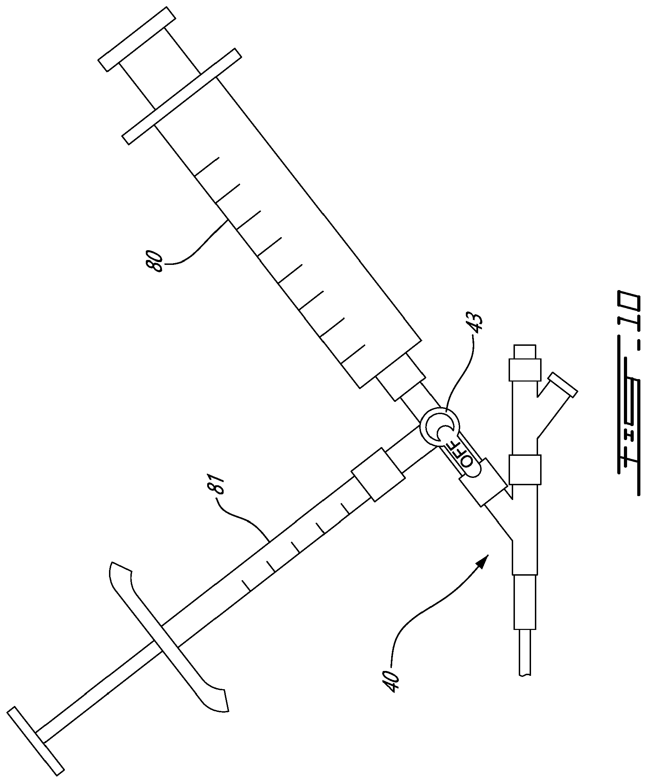

The distal shaft 30 may be loaded over the guidewire 11 as in 72 of the method 70. The catheter may be maneuvered under fluoroscopy until the and markers 34 are in the treatment area, whereby the foremost marker 34 is positioned distally to the thrombus. The balloon 23 may be inflated once the catheter is in the treatment area by turning the three-way valve 43 to put the vertical port to the OFF position thereby allowing the vacuum in the catheter to draw fluid from the syringe 80. After a given amount of time, three-way valve 43 may be actuated to put the proximal port to the OFF position. Under fluoroscopy or like imagining, optionally, the syringe 81 may incrementally inflate the balloon 23. Once the balloon 23 reaches the required occlusion diameter under fluoroscopy, the three-way valve 43 may be adjusted to put the distal port to the OFF position and maintain the inflation of the balloon 23 as in Fig. 10.

With the valve 43 in the position of Fig. 10, the catheter may also be used to deliver a pharmacologic agent by attaching a syringe with a desired infusion volume to the injection port (the free end of the central tubular portion 40A). The syringe may optionally have a flexible tube. For precaution, the plunger of the syringe may be pulled to withdraw any air bubbles. A continuous pressure may be applied on the plunger of the syringe to deliver the pharmacologic agent via the stylet 50 (Figs. 1 and 2). The syringe may be removed after use.

The aspiration catheter assembly may be used with aspiration accessories. The aspiration accessories may be primed by attaching an extension tube to an aspiration syringe containing 5 to 10 mL of heparinized saline, the extension tube having a stopcock that can be placed in an open position to flush the entire length of the extension tube; the stopcock may be closed after flushing and the plunger of the aspiration syringe may be pulled until reaching its full extension before being locked in place.

After the infusion, the stylet 50 may be removed. In a variant, this is achieved by removing the part of the catheter hub 40 including the branch portion 40C, thereby liberating the aspiration lumen 21 (Figs. 4 and 5)

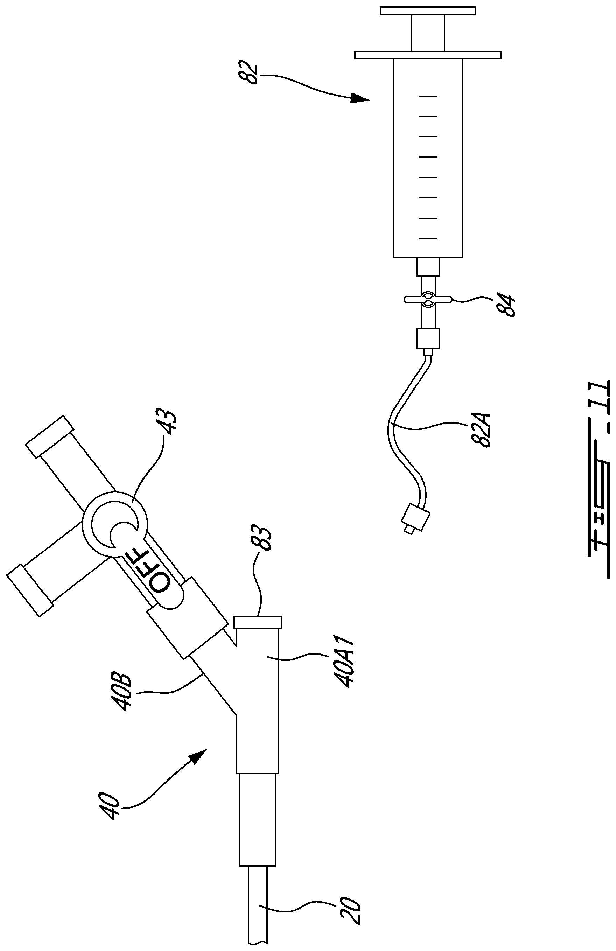

The catheter may be prepared for aspiration by turning the three-way valve 43 to put the vertical port in the OFF position; the plunger of the syringe may be pulled to deflate the balloon 23, which may be confirmed by fluoroscopy. The three-way valve 43 may then be rotated to put the distal port in the OFF position while maintaining the plunger in the pulled position. The two syringes may be removed after use. Then, as in Fig. 11, the catheter may be used to aspirate by connecting an optional extension tube 82A of an aspiration syringe 82 attached to the aspiration port 83 of the catheter. If present, a valve 84 on the extension line 82A or elsewhere may be opened. These steps may occur throughout some of the steps of the method 70 of Fig. 7.

The non-claimed method can be generally described as being for treating a thrombus (e.g., removing a thrombus) and may include percutaneously positioning a guide wire into an artery toward the thrombus; moving a distal shaft of a catheter along the guide wire until a tip of an infusion lumen of the distal shaft passes through the thrombus to a distal location, while an inflatable member and an aspiration lumen extending into a proximal shaft of the catheter are proximal to the thrombus; and infusing at least one pharmacologic agent distally to the thrombus via the infusion lumen.

Optionally, the non-claimed method may include inflating the inflatable member proximally to the thrombus the inflating may be performed prior to infusing at least one pharmacologic agent distally to the thrombus; performing an aspiration via the aspiration lumen to aspire the thrombus; positioning the catheter and passing of the tip are done by moving the catheter over a guide wire; moving the distal shaft of the catheter over the guide wire may include only the distal shaft being engaged onto the guide wire by a guide lumen thereof; infusing at least one pharmacologic agent includes infusing the pharmacologic agent in a tube of a stylet extending to the infusion lumen.

The catheter assembly comprises a distal shaft having a guide lumen and an infusion lumen; a proximal shaft connected to the distal shaft, the proximal shaft defining an aspiration lumen opening at or near the distal shaft, and an inflating lumen; an inflatable member at a distal end of the inflating lumen, the inflatable member configured to inflate upon receiving a fluid from the inflating lumen; a stylet tube extending along the aspiration lumen and in fluid communication with the infusion lumen, the stylet tube configured to supply the infusion lumen with a pharmacologic agent; wherein the catheter assembly is configured to be displaced percutaneously in an artery by the guide lumen moving along a guide wire.

Optionally, the catheter assembly may include a catheter hub at a proximal end of the proximal shaft. The catheter hub may be in fluid communication with the stylet tube, with the aspiration lumen and with the inflating lumen. The catheter hub may or may not have a port dedicated to the inflating lumen. A three-way valve on the port dedicated to the inflating lumen. Radio-opaque markers may be provided at or near opposed ends of the infusion lumen. The stylet is removable from the aspiration lumen prior to aspiration. Thus the aspiration lumen may be used for infusion (e.g., via the stylet or other tube) and for aspiration. The distal shaft has an aspiration port in fluid communication with the aspiration lumen. A proximal end of the guide lumen may be at the distal shaft.

The present disclosure also pertains to a non-claimed use of a catheter assembly, in the manner shown in the figures herein, the use of the catheter assembly for removing a thrombus from an artery, the use including: a guide wire for being percutaneously positioned into an artery toward the thrombus; a distal shaft of a catheter for being moved along the guide wire until a tip of an infusion lumen of the distal shaft passes through the thrombus to a distal location; an inflatable member on the catheter and in fluid communication with an inflating lumen of the catheter for inflating and blocking the artery proximally of the thrombus; an aspiration lumen in the catheter extending into a proximal shaft of the catheter to reach a position proximal to the thrombus, for aspiring the thrombus; and a stylet removably in the aspiration lumen of proximal shaft and in fluid communication with the infusion lumen for infusing at least one pharmacologic agent distally to the thrombus via the infusion lumen.

Numerous pharmacologic agents may be used for the infusion, the doses of which are selected on a case-by-case basis by appropriate personnel. Among the pharmacologic agents that can be used are: adenosine and adenosine receptor agonists, beta blockers, angiotensin-converting enzyme inhibitor, angiotensin-receptor blockers, aldosterone, calcium channel blockers (Verapamil, Diltiazem, Nifedipine), cyclosporin, calpain inhibitor, sodium-proton exchanger inhibitor, NO donor, cox-2 inhibitor, statins, TNF-alpha, endothelin receptor antagonists, antiplatelet, antithrombotic, erythropoietin, anti-leukocyte complement inhibitors, opioid, anesthetic, K_{ATP} "openers", insulin, thrombin and fragments, melatonin, H₂S, bradykinin, cellular therapy, gene therapy, with the proper catheter.

## Claims

1. A catheter assembly (10) comprising
a distal shaft (30) having a guide lumen (32);
a proximal shaft (20) connected to the distal shaft (30), the proximal shaft (20) defining an aspiration lumen (21) opening at or near the distal shaft (30), and an inflating lumen (22);
an inflatable member (23) at a distal end of the inflating lumen (22), the inflatable member (23) configured to inflate upon receiving a fluid from the inflating lumen (22);
wherein the catheter assembly (10) is configured to be displaced percutaneously in an artery by the guide lumen (32) moving along a guide wire (11);
**characterized in that**
the distal shaft (30) has an infusion lumen (31); and
a stylet tube (50) extends along the aspiration lumen (21) and is in fluid communication with the infusion lumen (31), the stylet tube (50) configured to supply the infusion lumen (31) with a pharmacologic agent.

2. The catheter assembly (10) according to claim 1, further including a catheter hub (40) at a proximal end of the proximal shaft (20).

3. The catheter assembly (10) according to claim 2, wherein the catheter hub (40) is in fluid communication with the stylet tube (50), with the aspiration lumen (21) and with the inflating lumen (22).

4. The catheter assembly (10) according to claim 3, wherein the catheter hub (40) has a port dedicated to the inflating lumen (22).

5. The catheter assembly (10) according to claim 4, including a three-way valve (43) on the port dedicated to the inflating lumen (22).

6. The catheter assembly (10) according to any one of claims 1 to 5, including radio-opaque markers (34) at or near opposed ends of the infusion lumen (31).

7. The catheter assembly (10) according to any one of claims 1 to 6, wherein the stylet (50) is removable from the aspiration lumen (21) prior to aspiration.

8. The catheter assembly (10) according to any one of claims 1 to 7, wherein the distal shaft (30) has an aspiration port (30A) in fluid communication with the aspiration lumen (21).

9. The catheter assembly (10) according to any one of claims 1 to 8, wherein a proximal end of the guide lumen (32) is at the distal shaft (30).

10. The catheter assembly (10) according to any one of claims 1 to 9, further including a guide wire (11).

## Patentansprüche

1. Katheteranordnung (10), die Folgendes umfasst
eine distale Welle (30) mit einem Führungslumen (32) ;
eine proximale Welle (20), die mit der distalen Welle (30) verbunden ist, wobei die proximale Welle (20) ein Aspirationslumen (21), das sich an oder in der Nähe der distalen Welle (30) öffnet und ein Aufblaslumen (22) definiert;
ein aufblasbares Element (23) an einem distalen Ende des Aufblaslumens (22), wobei das aufblasbare Element (23) dazu ausgelegt ist, nach Empfangen eines Fluids vom Aufblaslumen (22) aufgeblasen zu werden;
wobei die Katheteranordnung (10) dazu ausgelegt ist, durch das Führungslumen (32), das sich entlang eines Führungsdrahts (11) bewegt, perkutan in eine Arterie versetzt zu werden;
**dadurch gekennzeichnet, dass**
die distale Welle (30) ein Infusionslumen (31) aufweist; und
sich ein Sondenrohr (50) entlang des Aspirationslumens (21) erstreckt und mit dem Infusionslumen (31) in Fluidkommunikation steht, wobei das Sondenrohr (50) dazu ausgelegt ist, dass Infusionslumen (31) mit einem pharmakologischen Mittel zu versorgen.

2. Katheteranordnung (10) nach Anspruch 1, die ferner an einem proximalen Ende der proximalen Welle (20) eine Katheternabe (40) beinhaltet.

3. Katheteranordnung (10) nach Anspruch 2, wobei die Katheternabe (40) mit dem Sondenrohr (50), mit dem Aspirationslumen (21) und mit dem Aufblaslumen (22) in Fluidkommunikation steht.

4. Katheteranordnung (10) nach Anspruch 3, wobei die Katheternabe (40) einen Port aufweist, der für das Aufblaslumen (22) dediziert ist.

5. Katheteranordnung (10) nach Anspruch 4, die am Port, der für das Aufblaslumen (22) dediziert ist, ein Dreiwegeventil (43) beinhaltet.

6. Katheteranordnung (10) nach einem der Ansprüche 1 bis 5, die an oder in der Nähe von gegenüberliegenden Enden des Aufblaslumens (31) röntgendichte Marker (34) beinhaltet.

7. Katheteranordnung (10) nach einem der Ansprüche 1 bis 6, wobei die Sonde (50) vor der Aspiration aus dem Aspirationslumen (21) entfernbar ist.

8. Katheteranordnung (10) nach einem der Ansprüche 1 bis 7, wobei die distale Welle (30) einen Aspirationsport (30A) aufweist, der mit dem Aspirationslumen (21) in Fluidkommunikation steht.

9. Katheteranordnung (10) nach einem der Ansprüche 1 bis 8, wobei sich ein proximales Ende des Führungslumens (32) an der distalen Welle (30) befindet.

10. Katheteranordnung (10) nach einem der Ansprüche 1 bis 9, die ferner einen Führungsdraht (11) beinhaltet.

## Revendications

1. Un ensemble de cathéter (10) comprenant
une tige distale (30) comportant une lumière de guidage (32);
une tige proximale (20) reliée à la tige distale (30), la tige proximale (20) définissant une lumière d'aspiration (21) s'ouvrant au niveau ou à proximité de la tige distale (30), et une lumière de gonflage (22) ;
un élément gonflable (23) à une extrémité distale de la lumière de gonflage (22), l'élément gonflable (23) étant configuré pour se gonfler lorsqu'il reçoit un fluide provenant de la lumière de gonflage (22) ;
l'ensemble de cathéter (10) étant configuré pour être déplacé par voie percutanée dans une artère par la lumière de guidage (32) se déplaçant le long d'un fil guide (11) ;
**caractérisé en ce que**
la tige distale (30) a une lumière de perfusion (31) ; et
un tube de stylet (50) s'étend le long de la lumière d'aspiration (21) et est en communication fluidique avec la lumière de perfusion (31), le tube de stylet (50) étant configuré pour alimenter la lumière de perfusion (31) avec un agent pharmacologique.

2. L'ensemble de cathéter (10) selon la revendication 1, comprenant en outre un raccord de cathéter (40) à une extrémité proximale de la tige proximale (20).

3. L'ensemble de cathéter (10) selon la revendication 2, dans lequel le raccord de cathéter (40) est en communication fluidique avec le tube de stylet (50), avec la lumière d'aspiration (21) et avec la lumière de gonflage (22).

4. L'ensemble de cathéter (10) selon la revendication 3, dans lequel le raccord de cathéter (40) comporte un orifice dédié à la lumière de gonflage (22).

5. L'ensemble de cathéter (10) selon la revendication 4, comprenant une vanne à trois voies (43) sur l'orifice dédié à la lumière de gonflage (22).

6. L'ensemble de cathéter (10) selon l'une quelconque des revendications 1 à 5, comprenant des marqueurs radio-opaques (34) au niveau ou à proximité des extrémités opposées de la lumière de perfusion (31).

7. L'ensemble de cathéter (10) selon l'une quelconque des revendications 1 à 6, dans lequel le stylet (50) est apte à être retiré de la lumière d'aspiration (21) avant l'aspiration.

8. L'ensemble de cathéter (10) selon l'une quelconque des revendications 1 à 7, dans lequel la tige distale (30) comporte un orifice d'aspiration (30A) en communication fluidique avec la lumière d'aspiration (21).

9. L'ensemble de cathéter (10) selon l'une quelconque des revendications 1 à 8, dans lequel une extrémité proximale de la lumière de guidage (32) se trouve au niveau de la tige distale (30).

10. L'ensemble de cathéter (10) selon l'une quelconque des revendications 1 à 9, comprenant en outre un fil guide (11).
